# EUROPEAN PATENT APPLICATION

(11) **EP 0 617 008 A1**
(43) Date of publication of application: **28.09.1994**
(21) Application number: 94610008.8
(22) Date of filing: 19.01.1994
(51) Int. Cl.: C07C 229/76, C07C 229/16, C07C 227/18, C07F 9/38, C05D 9/02

(54) **Iron chelate solutions**

(30) Priority: 20.01.1993 DK 69/93
(71) Applicant: AMINKEMI AB, S-251 09 Helsingborg (SE)
(72) Inventor: Hjelte, Nils Sixten, S-257 30 Rydebäk (SE)
(74) Representative: Plougmann, Vingtoft & Partners A/S

(57) **Abstract**

A method for the preparation of an aqueous solution of iron polyprotic acid chelate complexes, comprising reacting a solution of a mixture of a first polyprotic acid or a salt thereof, said first acid being capable of chelating iron, and a second polyprotic acid or a salt thereof, said second acid being capable of chelating iron and being different from the first acid, with iron in the presence of an oxidizing agent, with the proviso that at least part of the carboxylic acid groups in the first and second acids is present in salt form and at least part of the carboxylic acid groups in the first and second acids is present in acid form. Preferably, the first and second polyprotic acids may be selected from ethylenediaminetetraacetic acid (EDTA), N-hydroxyethyl-ethylenediaminetriacetic acid (HEDTA), and diethylenetriaminepentaacetic acid (DTPA), and the iron is selected from Fe-powder, Fe(OH)₂ and FeCO₃. Part of the polyprotic acid groups in the first and second acids is present in the salt form, the salt being selected from ammonium and alkali metal ions, in particular K⁺, Na⁺ and mixtures thereof. The method makes it possible to obtain aqueous solutions containing high contents of iron, such as up to approximately 7% by weight of iron, for use as a plant nutrient, mainly in soil-free plant culturing.

## Description

### FIELD OF THE INVENTION

The present invention relates to a method for preparing solutions of iron chelates for use as a plant nutrient, mainly in soil-free culturing of plants, as well as such iron chelate solutions *per* *se*.

### BACKGROUND OF THE INVENTION

During the last decades, greenhouse gardening has become an industry of great importance in the supply of vegetables, fruits and flowers outside the normal season of these crops. In order to ensure a stable supply of vegetables, fruits, flowers, etc. whenever the demands are present, extensive greenhouse gardening has been developed using very sophisticated methods. In the so-called soil-free culture, i.e. growing plants on fibrous supports saturated with aqueous nutrient solutions instead of soil, all the nutrients (including micronutrients such as various metal ions) which are normally absorbed by the plant from the soil must be supplied artificially through the aqueous nutrient medium in order to ensure the growth of the plants.

One of the very important nutrient components is iron, without which crop plants are not able to grow properly. For many years iron has been supplied in the form of iron chelates which have the advantage of keeping the iron in solution in the presence of iron precipitating ions in the nutrient solution. However, it has previously not been possible to obtain iron chelate stock solutions with concentrations higher than about 3% by weight of iron.

A typical process for producing aqueous iron chelate solutions has been by reacting carboxylated and hydroxyalkylated amines, typically EDTA (ethylenediaminetetraacetic acid as the tetrasodium salt), with iron(II) or iron(III) compounds, typically FeCl₃ (ferric chloride) or FeSO₄ (ferrous sulfate).

Consequently, the products of such processes have the further disadvantage that they also contain substantial amounts of undesired ionic salts, in particular salts containing chloride and sulphate ions, e.g. NaCl or Na₂SO₄, which cannot be absorbed by the plants but will instead accumulate in the nutrient solution. Due to environmental considerations, the authorities of many countries do not allow direct discharge of nutrient solutions into sewage systems and natural bodies of water which necessitates the recirculation of the nutrient solutions. In the end, however, following prolonged addition of iron chelate solution and recirculation of the nutrient solution, the concentration of salts in the nutrient medium will be so high that the plants will begin to be negatively affected due to the phytotoxic properties of many salts. One attempt at eliminating the problem of salts involved a process in which EDTA in acid form was reacted with iron powder, but this process suffers from the drawback that EDTA in acid form is not very soluble in water, which in turn means that the reaction rate is very low since only a low concentration of EDTA is able to react with the iron powder, the entire reaction thus essentially being a three-phase system.

### SUMMARY OF THE INVENTION

In a first aspect, the invention relates to a method for the preparation of an aqueous solution of iron polyprotic acid chelate complexes, said method comprising reacting a solution of a mixture of a first polyprotic acid or a salt thereof, said first acid being capable of chelating iron, and a second polyprotic acid or a salt thereof, said second acid being capable of chelating iron and being different from the first acid, with iron in the presence of an oxidizing agent, with the proviso that at least part of the acid groups in the first and second acids is present in salt form and at least part of the acid groups in the first and second acids is present in acid form. By the method according to the invention, the above-discussed problems have been alleviated since for example chloride salts are not formed.

In a second aspect, the invention concerns an aqueous solution comprising iron polyprotic acid chelate complexes, said complexes being formed between on the one hand Fe(III) cations or mixtures of Fe(II) and Fe(III) cations and on the other hand a mixture of anions of a first polyprotic acid, said first acid being capable of chelating iron, and anions of a second polyprotic acid, said second acid being capable of chelating iron and being different from the first acid.

### DETAILED DESCRIPTION OF THE INVENTION

In the present context, the term "polyprotic acid" is intended to mean any organic acid containing two or more acid groups (i.e. groups capable of splitting off at least one hydrogen ion) in the molecule, such as from two to five acid groups.

Also, in the present context, the phrase "capable of chelating iron" as applied to the first and second polyprotic acids above means that one molecule of the polyprotic acid is able to form a stable coordination complex with one iron ion (Fe(II) or Fe(III)) and occupying at least 2 or more, preferably 3 or more, in particular 4 or more, of the 6 available octahedrally arranged ligand positions on Fe(II) and Fe(III) without requiring the presence of further molecules of chelating agent.

Furthermore, in the present context, the phrase "at least part of the acid groups in the first and second acids is present in salt form and at least part of the acid groups in the first and second acids is present in acid form" means that some of the total number of acid groups present in the amount of the first or the second polyprotic acid used are present in acid form while the remaining acid groups present in the amount of the same polyprotic acid are present in salt form. Thus, since this proviso applies to the solution of the mixture of the two polyprotic acids, this could be fulfilled on the one hand by using as for example the first polyprotic acid, an amount of that acid in acid form mixed with an amount of that acid in pure or partial salt form, or on the other hand by using solely a partial salt (i.e. that some of the acid groups in each molecule are in acid form and the remainder are in salt form) of the acid in question. However, the proviso could also be fulfilled by using the pure acid of one polyprotic acid and a complete or partial salt of the other polyprotic acid.

One of the important features of the invention is that, contrary to the prior art, two different chelating agents (the polyprotic acids mentioned above) are used in the manufacturing method, and it is contemplated that this is at least in part responsible for the fact that concentrations of above 3% by weight and up to 6% by weight or more of iron in the resulting solution can be attained. Furthermore, this feature is also believed to help increase the rate of the reaction with the iron when used in metallic form.

Examples of acid groups which may be present in the molecule of the polyprotic acid are carboxylic, phosphonic, and sulfonic acid groups. It is preferred, however, that the acid groups in the first and second polyprotic acid molecule are carboxylic acid groups (COOH), phosphonic acid groups (PO₃H₂) or combinations thereof.

In a preferred embodiment of the method of the invention, the first and the second polyprotic acids are selected from compounds of the general formula I 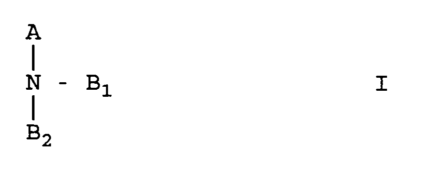
and the general formula II 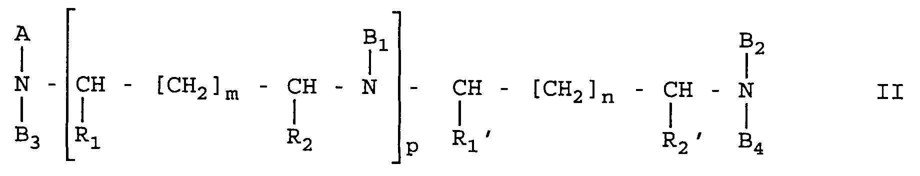
in which A is CH₂COOH, CH₂PO₃H₂, CH₂CH₂OH or CH₂CH₂CH₂OH; B₁, B₂, B₃ and B₄ independently are CH₂COOH or CH₂PO₃H₂; m and n each independently designate an integer of 0 or 1; p designates an integer of 0 or 1; the groups R₁, R₁', R₂ and R₂', each independently designate H or CH₃, or, when m designates 0, the groups R₁ and R₂ together may also designate -(CH₂)₄-, or, when n designates 0, the groups R₁' and R₂' together may also designate -(CH₂)₄-.

In order to ensure a suitable reaction rate, it is preferred that at least 20 mole percent and not more than 70 mole percent of the polyprotic acid groups in the solution of the first and second acids are present in the salt form, in particular that at least 30 mole percent and not more than 60 mole percent, especially at least 40 mole percent and not more than 50 mole percent, of the polyprotic acid groups in the solution of the first and second acids are present in the salt form.

Typical, but not exhaustive, examples of compounds of the general formulas I and II are the following:
Tri(carboxymethyl)amine,
ethylenediaminetetraacetic acid (EDTA),
N,N,N',N'-tetracarboxymethyl-1,2-diamino-propane,
N,N,N',N'-tetracarboxymethyl-1,2-diamino-cyclohexane (cis/trans),
N,N,N',N'',N''-pentacarboxymethyl-N'-(2-aminoethyl)-1,2-diamino-ethane,
N,N,N',N'',N''-pentacarboxymethyl-N'-(3-aminopropyl)-1,2-diamino-ethane,
N,N,N',N'',N''-pentacarboxymethyl-N'-(2-aminopropyl)-1,2-diamino-propane,
N,N,N',N'',N''-pentacarboxymethyl-N'-(3-aminopropyl)-1,3-diamino-propane,
N,N,N',N'',N''-pentacarboxymethyl-N'-(2-aminopropyl)-1,2-diamino-ethane,
di(carboxymethyl)-(2-hydroxyethyl)amine,
N,N,N'-tricarboxymethyl-N'-(2-hydroxyethyl)-1,2-diaminoethane [N-hydroxyethyl-ethylenediaminetriacetic acid (HEDTA)],
N,N,N'-tricarboxymethyl-N'-(2-hydroxyethyl)-1,2-diaminopropane,
N,N,N'-tricarboxymethyl-N'-(2-hydroxyethyl)-1,2-diaminocyclohexane (cis/trans),
N,N,N',N''-tetracarboxymethyl-N'-(2-aminoethyl)-N''-(2-hydroxyethyl)-1,2-diamino-ethane,
N,N,N',N''-tetracarboxymethyl-N'-(3-aminopropyl)-N''-(2-hydroxyethyl)-1,2-diamino-ethane,
N,N,N',N''-tetracarboxymethyl-N'-(2-aminopropyl)-N''-(2-hydroxyethyl)-1,2-diamino-propane,
N,N,N',N''-tetracarboxymethyl-N'-(3-aminopropyl)-N''-(2-hydroxyethyl)-1,3-diamino-propane,
N,N,N',N''-tetracarboxymethyl-N'-(2-aminopropyl)-N''-(2-hydroxyethyl)-1,2-diamino-ethane,
di(carboxymethyl)-(3-hydroxypropyl)amine,
N,N,N'-tricarboxymethyl-N'-(3-hydroxypropyl)-1,2-diaminoethane,
N,N,N'-tricarboxymethyl-N'-(3-hydroxypropyl)-1,2-diaminopropane,
N,N,N'-tricarboxymethyl-N'-(3-hydroxypropyl)-1,2-diaminocyclohexane (cis/trans),
N,N,N',N''-tetracarboxymethyl-N'-(2-aminoethyl)-N''-(3-hydroxypropyl)-1,2-diamino-ethane,
N,N,N',N''-tetracarboxymethyl-N'-(3-aminopropyl)-N''-(3-hydroxypropyl)-1,2-diamino-ethane,
N,N,N',N''-tetracarboxymethyl-N'-(2-aminopropyl)-N''-(3-hydroxypropyl)-1,2-diamino-propane,
N,N,N',N''-tetracarboxymethyl-N'-(3-aminopropyl)-N''-(3-hydroxypropyl)-1,3-diamino-propane,
N,N,N',N''-tetracarboxymethyl-N'-(2-aminopropyl)-N''-(3-hydroxypropyl)-1,2-diamino-ethane,
ethylenediaminetetra(methylenephosphonic acid),
hexamethylenediaminetetra(methylenephosphonic acid),
diethylenetriaminepenta(methylenephosphonic acid),
as well as partial or complete salts thereof.

In a particularly preferred embodiment of the method of the invention, the first and second polyprotic acids are selected from ethylenediaminetetraacetic acid (EDTA), N-hydroxyethylethylenediaminetriacetic acid (HEDTA), and diethylenetriaminepentaacetic acid (DTPA). In an especially preferred embodiment of the invention, the first polyprotic acid is ethylenediaminetetraacetic acid (EDTAH₄) and the second polyprotic acid is the trisodium salt of N-hydroxyethyl-ethylenediaminetriacetic acid (HEDTANa₃).

The compounds of the general formula I or II are either known or may be prepared by methods analogous to those used for preparing known compounds. For example, the methylphosphonic acid derivatives mentioned in the list above are available from Monsanto under the trade name Dequest™ series 2040, 2050 and 2060.

Thus, a CH₂COOH group in A and B₁₋₄ is typically introduced by reacting the corresponding unsubstituted amine with an equimolar mixture of sodium or potassium cyanide and formaldehyde in an aqueous solution, the number of moles of cyanide being at least equal to the number of CH₂COOH groups to be introduced. In a typical process, for example, a 25% ammonia solution contained in a reaction vessel equipped with a stirrer, heating facilities and a cooling funnel connected to an ammonia scrubber system is reacted with an aqueous solution of sodium cyanide and an aqueous solution of formaldehyde by gradually adding the cyanide and the formaldehyde solutions and while gradually increasing the temperature to remove the ammonia formed in the process. Acidification with a suitable acid such as hydrochloric acid is added to precipitate tri(carboxymethyl)amine.

Likewise, a CH₂PO₃H₂ group A and B₁₋₄ is typically introduced by reacting the corresponding amine with the relevant amount (determined by the number of CH₂PO₃H₂ groups to be inserted) of an equimolar mixture of formaldehyde and phosphorous acid in an aqueous medium at elevated temperature, see e.g. US 4,671,934.

In the method according to the invention, the salts of the first polyprotic acid and the salts of the second polyprotic acid, are preferably selected from ammonium and alkali metal salts, in particular Na⁺, K⁺ and mixtures thereof.

The iron added to the aqueous solution during the method according to the invention may be in the form of Fe-powder, Fe(OH)₂ or FeCO₃, preferably Fe-powder.

One of the important features of the present invention which also sets it apart from the known art is that by the method described, it is possible to obtain aqueous solutions that contain more than 3% by weight of iron, such as at least 4% by weight of iron, preferably at least 5% by weight of iron, in particular at least 6% by weight of iron, thereby substantially reducing the bulk and hence the transportation costs of such solutions.

When using Fe-powder in the method according to the invention, a suitable iron powder product is one in which 60% by weight or more of the particles have a size in the range of 45 to 150 µm.

The oxidizing agent added according to the invention is one which is capable of oxidizing Fe(II) to Fe(III) and may e.g. be selected from O₂, H₂O₂ and nitric acid. In a preferred embodiment of the invention, the oxidizing agent is gaseous O₂ in particular O₂ contained in atmospheric air. However, any combination of O₂ with inert, non-oxidizing compounds can be used for the purpose of the invention.

The pH of the final aqueous solution is preferably adjusted to a value in the range of 5-7.5, more preferably in the range of 5.5-7.

As indicated above, a second aspect of the present invention relates to an aqueous solution comprising iron polyprotic acid chelate complexes, said complexes being formed between on the one hand Fe(III) cations or mixtures of Fe(II) and Fe(III) cations and on the other hand a mixture of anions of a first polyprotic acid, said first acid being capable of chelating iron, and anions of a second polyprotic acid, said second acid being capable of chelating iron and being different from the first acid. Such a solution is preferably essentially free from halide ions.

In a preferred embodiment of the solution of the invention, the first and second polyprotic acids contain carboxylic acid groups, phosphonic acid groups or a combination of carboxylic and phosphonic acid groups. It is particularly preferred that the first and second polyprotic acids are selected from compounds of the general formulas I and II defined above, especially such in which B₁, B₂, B₃ and B₄ are CH₂COOH. Typical examples of the first and second polyprotic acids, the anions of which may be used in the solution of the invention, are those listed above as examples of acids of the general formulas I and II. Examples of especially preferred polyprotic acids, from which the first and second polyprotic acids are selected, are ethylenediaminetetraacetic acid (EDTA), N-hydroxyethyl-ethylenediaminetriacetic acid (HEDTA), and diethylenetriaminepentaacetic acid (DTPA).

In the solutions of the invention, the total concentration of iron polyprotic acid chelate complexes is preferably chosen so that the solution contains at least 3% by weight of iron, preferably 3-6% by weight, more preferably 4-6% by weight, in particular 5-6% by weight, such as approximately 6% by weight of iron.

The pH of the solution according to the invention is preferably in the range of 5-7.5, in particular in the range of 5.5-7.

The solution according to the invention may be used in a manner known per se for supplying iron to nutrient solutions used in soil-free agriculture. Thus, the concentration of chelated iron in the finished nutrient solution is typically in the range of 10-20 µM, preferably in the range of 12-18 µM, in particular about 15 µM.

The invention is further illustrated by the following, non-limiting examples.

### EXAMPLE 1

Preparation of iron chelates comprising HEDTA and EDTA

996 kg of a 39.7% w/w aqueous solution of technical HEDTANa₃ and 560 litres of water was placed in an open kettle provided with heating/cooling coils, a stirrer and a coil for bubbling air through the solution. While stirring and heating to 60-70°C, 400 kg of 99.9% EDTAH₄ was added and dissolved. 135 kg of iron powder (particle size distribution: 0.5% w/w 150-212 µm; 23.1% w/w 109-150 µm; 31.9% w/w 75-106 µm; 28.6% w/w 45-75µm; 15.9% w/w <45 µm) was added over a 3 hour-period while the mixture was gradually heated to 90°C over 2 hours. When the solution reached about 60°C, air was bubbled through the solution. The iron powder was completely dissolved and chelated after about 6 hours.

The solution was chilled and sufficient water was added to give a solution with a specific gravity of about 1.3 at 20°C. After filtration, a clear solution was obtained containing about 6% by weight of chelated iron mainly in the trivalent form.

### EXAMPLE 2

Preparation of a 6% iron chelate solution from iron carbonate

An aqueous solution containing iron carbonate (FeCO₃) and a 0.54/0.56 mole mixture of HEDTANa₃ and EDTAH₄ was heated to the boiling point. The iron carbonate was added in a 50% molar excess over the chelating agents. Air was bubbled through the solution, and after 3 hours of boiling the aqueous solution was cooled and filtered. Analysis of the filtrate for iron content indicated that 85% of the chelating agents had been converted into iron chelate. The aqueous solution was adjusted with water to contain 6% by weight of iron.

### EXAMPLE 3

To a mixture of 175.2 g of a 43.7% w/w aqueous solution of HEDTAK₃ and 125 g of water was added 191.6 g EDTAH₄, and the mixture was heated to 95°C. 65 g of iron powder (particle size distribution: 0.5% w/w 150-212 µm; 23.1% w/w 109-150 µm; 31.9% w/w 75-106 µm; 28.6% w/w 45-75µm; 15.9% w/w <45 µm) was added and air bubbled through the mixture for 5 hours while maintaining the temperature at 95°C. The mixture was cooled and filtered (to remove i.a. a small amount of unreacted iron powder) which gave 910 g of a solution containing 6.9% by weight of chelated iron. Following dilution with water to an iron content of 6% by weight, a solution was obtained having a pH of 7.1 and a specific gravity at 20°C of 1.313 g/cm³. Storage of the latter solution at 4°C for 4 weeks did not result in any crystal formation.

### EXAMPLE 4

This example illlustrates the use of an aqueous solution containing chelated iron in the production of cucumbers.

1.4 kg of the product of Example 1 containing 6% iron as chelated iron was mixed with the following components:
- Nitric acid, 38%: 42.6 kg
- Potassium hydroxide liq.: 28.8 kg
- Calcium nitrate liq.: 128.0 kg
- Ammonium nitrate: 19.5 kg

This solution was fed into the irrigation system of a green-house in which cucumber were grown. At the same time, a solution containing the following components in calculated amounts was also fed into the irrigation system:
Nitric acid
Phosphoric acid
Sulphuric acid
Potassium hydroxide
Magnesium sulphate
Magnesium nitrate
Manganese sulphate
Zinc sulphate
Sodium borate
Copper sulphate
Sodium molybdate

The two solutions are added at such a rate that a concentration of chelated iron of 15 µM is achieved, and at the same time a concentration of phosphate of 1.25 mM is achieved.

By the method described above, no precipitation of any salts was observed and the iron content available to the plants was analyzed, the results giving the expected values. The plants were kept on this regime for 3 months resulting in healthy growth and deep green leaves, showing that iron had been available to the plants.

## Claims

1. A method for the preparation of an aqueous solution of iron polyprotic acid chelate complexes, comprising reacting a solution of a mixture of a first polyprotic acid or a salt thereof, said first acid being capable of chelating iron, and a second polyprotic acid or a salt thereof, said second acid being capable of chelating iron and being different from the first acid, with iron in the presence of an oxidizing agent, with the proviso that at least part of the acid groups in the first and second acids is present in salt form and at least part of the acid groups in the first and second acids is present in acid form.

2. A method according to claim 1, in which the first and second polyprotic acids contain carboxylic acid groups, phosphonic acid groups or a combination of carboxylic and phosphonic acid groups.

3. A method according to claim 1 or 2, in which the first and the second polyprotic acids are selected from compounds of the general formula I 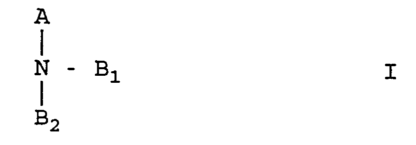 and the general formula II 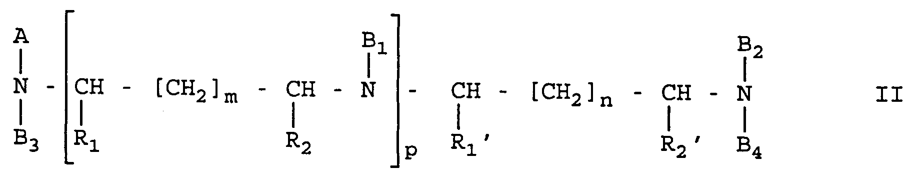 in which A is CH₂COOH, CH₂PO₃H₂, CH₂CH₂OH or CH₂CH₂CH₂OH; B₁, B₂, B₃ and B₄ independently are CH₂COOH or CH₂PO₃H₂; m and n each independently designate an integer of 0 or 1; p designates an integer of 0 or 1; the groups R₁, R₁', R₂ and R₂' each independently designate H or CH₃, or, when m designates 0, the groups R₁ and R₂ together may also designate -(CH₂)₄-, or, when n designates 0, the groups R₁' and R₂' together may also designate -(CH₂)₄-.

4. A method according to any of claims 1-3 in which at least 20 mole percent and not more than 70 mole percent of the polyprotic acid groups in the solution of the first and second acids are present in the salt form.

5. A method according to claim 4 in which at least 30 mole percent and not more than 60 mole percent of the polyprotic acid groups in the solution of the first and second acids are present in the salt form.

6. A method according to claim 5 in which at least 40 mole percent and not more than 50 mole percent of the polyprotic acid groups in the solution of the first and second acids are present in the salt form.

7. A method according to any of claims 3-6 in which A is CH₂COOH, CH₂CH₂OH or CH₂CH₂CH₂OH, and B₁, B₂, B₃ and B₄ are CH₂COOH.

8. A method according to any of the preceding claims in which the first and second polyprotic acids are selected from ethylenediaminetetraacetic acid (EDTA), N-hydroxyethyl-ethylenediaminetriacetic acid (HEDTA), and diethylenetriaminepentaacetic acid (DTPA).

9. A method according to any of the preceding claims in which the cation of the salt form of the first and second polyprotic acids is selected from ammonium and alkali metal ions, in particular K⁺, Na⁺ and mixtures thereof.

10. A method according to claim 8 in which the ethylenediaminetetraacetic acid or salts thereof have the general formula EDTAX₄, each X being independently selected from H, Na, K and NH₄.

11. A method according to claim 8 in which the hydroxyethylethylenediaminetriacetic acid or salts thereof have the general formula HEDTAX₃, each X being independently selected from H, Na, K and NH₄.

12. A method according to claim 8 in which the diethylenetriaminepentaacetic acid or salts thereof have the general formula DTPAX₅, each X being independently selected from H, Na, K and NH₄.

13. A method according to any of the preceding claims in which the iron is added to the aqueous solution in the form of Fe-powder, Fe(OH)₂ or FeCO₃.

14. A method according to any of the preceding claims in which the oxidizing agent is selected from O₂, hydrogen peroxide and nitric acid.

15. A method according to any of the preceding claims in which the solution of the mixture of the first polyprotic acid and the second polyprotic acid is prepared from EDTAH₄ and HEDTANa₃.

16. A method according to any of the preceding claims in which the solution obtained contains at least 4% by weight of iron, preferably at least 5% by weight of iron, in particular at least 6% by weight of iron.

17. A method according to any of the preceding claims in which the pH of the final aqueous solution is adjusted to at least 5 and not more than 7.5, preferably to at least 5.5 and not more than 7.

18. An aqueous solution comprising iron polyprotic acid chelate complexes, said complexes being formed between on the one hand Fe(III) cations or mixtures of Fe(II) and Fe(III) cations and on the other hand a mixture of anions of a first polyprotic acid, said first acid being capable of chelating iron, and anions of a second polyprotic acid, said second acid being capable of chelating iron and being different from the first acid.

19. A solution according to claim 18, in which the first and second polyprotic acids contain carboxylic acid groups, phosphonic acid groups or a combination of carboxylic and phosphonic acid groups.

20. An aqueous solution according to claim 18 or 19 in which the first and the second polyprotic acids are selected from compounds of the general formula I 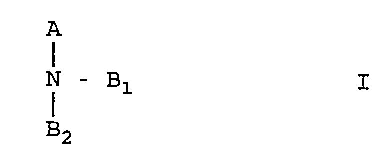 and the general formula II 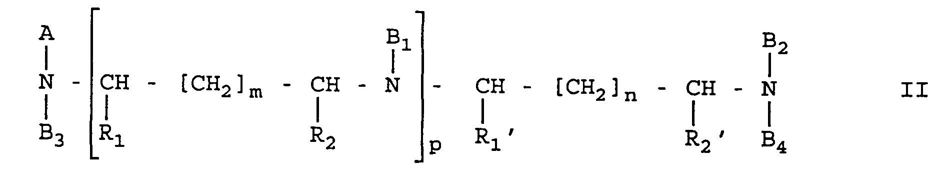 in which A is CH₂COOH, CH₂PO₃H₂, CH₂CH₂OH or CH₂CH₂CH₂OH; B₁, B₂, B₃ and B₄ independently are CH₂COOH or CH₂PO₃H₂; m and n each independently designate an integer of 0 or 1; p designates an integer of 0 or 1; the groups R₁, R₁', R₂ and R₂' each independently designate H or CH₃, or, when m designates 0, the groups R₁ and R₂ together may also designate -(CH₂)₄-, or, when n designates 0, the groups R₁' and R₂' together may also designate -(CH₂)₄-.

21. A solution according to claim 20 in which A is CH₂COOH, CH₂CH₂OH or CH₂CH₂CH₂OH, and B₁, B₂, B₃ and B₄ are CH₂COOH.

22. A solution according to any of claims 18-21 in which the first and second polyprotic acids are selected from ethylenediaminetetraacetic acid (EDTA), N-hydroxyethyl-ethylenediaminetriacetic acid (HEDTA), and diethylenetriaminepentaacetic acid (DTPA).

23. An aqueous solution according to any of claims 18-22 in which the total concentration of iron polyprotic acid chelate complexes is such that the solution contains at least 3% by weight of iron, preferably 3-6% by weight, more preferably 4-6% by weight, in particular 5-6% by weight, such as approximately 6% by weight of iron.

24. An aqueous solution according to any of claims 18-23 in which the pH is in the range of 5-7.5, preferably in the range of 5.5-7.
